# EUROPEAN PATENT SPECIFICATION

(11) **EP 0 344 913 B1**
(45) Date of publication and mention of the grant of the patent: **24.09.1997**
(21) Application number: 89304324.0
(22) Date of filing: 28.04.1989
(51) Int. Cl.: A61L 15/28, A61F 13/00, D04H 1/42, D04H 1/44

(54) **Alginate wound dressing of good integrity**
Alginatewundverband mit guter Integrität
Pansement d'alginate de bonne intégrité

(30) Priority: 31.05.1988 US 200711
(43) Date of publication of application: 06.12.1989
(73) Proprietor: MINNESOTA MINING AND MANUFACTURING COMPANY, St. Paul, Minnesota 55133-3427 (US)
(72) Inventor: Cole, Susan M., P.O.Box 33427, St. Paul, MN 55133-3427 (US); Nelson, David L., P.O.Box 33427, St. Paul, MN 55133-3427 (US)
(74) Representative: Baillie, Iain Cameron

(56) References cited:
- WO-A-80/02300
- US-A- 3 485 706
- US-A- 4 704 113

## Description

U.K. Pat. Spec. No. 653,341 (Bonniksen) which was published May 16, 1951 says: "The use of calcium alginate materials, made up into wool, gauze, foam, and the like, in surgery is now well known. They are used, because of their absorbability in the body, to control hemorrhage, fill 'dead space' after the removal of organs or massive tissue, act as tissue isolating films, and externally as burn, ulcer and wound covers. They fill a recognized and important need, their properties having been described in detail by George Blaine in the Annals of Surgery for January, 1947" (page 2, lines 5-23). The Bonniksen specification further says that when a calcium alginate dressing is placed on an external wound, it swells in and is very slowly dissolved by the body fluids.

U.K. Pat. Spec. No. 1,394,741 (Franklin et al.) which was published May 21, 1975 says that calcium alginate materials have long been used as hemostatic surgical dressings, usually after being knitted into gauze and then replacing part of the calcium content by a more soluble cation such as sodium. Soluble cations are said to enable the alginate to disperse more readily in body fluids and tissues. A knitted alginate is currently being marketed as Ultraplast™ styptic gauze wound dressing by Wallace, Cameron & Co. Ltd., Glascow, Scotland.

U.S. Pat. No. 4,562,110 (Tong) says that the knitting of the dressing of the Franklin specification "has usually involved handling problems and has been carried out batchwise which is rather inefficient and uneconomic resulting in a costly and wasteful production process" (col. 1, lines 38-48). In the invention of the Tong patent, calcium alginate fiber material is first converted into the sodium/calcium mixed salt form, dried, and then "made up into a tow suitable for swab production or alginate wool or into the form of a non-woven wadding suitable for use as a medical or surgical dressing" (col. 1, lines 49-58).

The Tong patent discusses a process for making an alginate dressing as disclosed in International Pat. Appl. No. PCT/GB30/00066 (Courtaulds Ltd.) in which a tow of "calcium alginate fibres or filaments is passed in a flow of water through a spreading device, such as a device with a 'fish tail' outlet, and the spread band or sheet produced is fed forwards and deposited on a liquid permeable conveyor, such as Fourdrinier wire mesh conveyor, moving at a slower speed so that the fibres are overlaid in a substantially uniform layer or sheet forming a web which is then dried to provide a unitary non-woven alginate fabric. As a result of the overlaying, the fibres become crimped or looped and cross over each other in the web so that a parallel orientation thereof is destroyed" (col. 2, lines 40-59). This produces "a dried web of intersecting fibres or filaments bonded together at their cross-over points to provide a strong unitary structure" (col. 3, lines 18-23).

The Tong patent says that the process of the Courtaulds PCT application produces "a relatively harsh fabric likely to be very stiff and to have poor handling qualities, especially if one attempts to make up such webs into thick or multilayer fabrics" (col. 5, lines 7-15). The invention of the Tong patent concerns a process similar to that of the Courtaulds PCT application except including "at least one controlled treatment step which is effective to eliminate or reduce bonding of the overlaid fibres at their points of contact or intersection" (col. 4, lines 26-33).

U.S. Pat. No. 4,421,583 (Aldred et al.) corresponds to the Courtaulds PCT application.

Although it is not known whether alginate fabrics are being manufactured in accordance with any of the above-discussed processes, a carded web of alginate fibers is being marketed as Steriseal Sorbsan™ surgical dressing by N.I. Medical, Redditch, Worcestershire, England, and a carded and needle-tacked web of alginate fibers is being marketed as Kaltostat™ haemostatic wound dressing by Cair Ltd., Aldershot, Hatt, England. Alginate tow is also marketed by each of those companies under the same tradenames for use as wound dressings, and especially for surgical packing.

Except for the knitted Ultraplast™ and the needle-tacked Kaltostat™ wound dressings, each of the aforementioned commercial alginate products has poor integrity and hence is difficult to handle. However, the needle-tacked Kaltostat™ wound dressing has a relatively high basis weight, about 160 g/m², and is not as supple as would be desired for most applications. It is assumed that the needle-tacked Kaltostat™ dressing is not offered at lower basis weights, because it then would be rather weak. Additionally, because alginate fibers are highly absorbent, dressings based on high basis weight webs of the fibers would be more likely to desiccate a wound if applied to the wound in a dry condition. The manufacturer of the needle-tacked Kaltostat™ dressing avoids this problem by recommending that the dressing be moistened before application to the wound.

Except for the knitted Ultraplast™ wound dressing, the commercial alginate wound dressings mentioned above are weak and tend to shed fibers. Because of their weakness, skill is required to apply the dressing to wounds, and handling problems are aggravated when ones fingers are not completely dry.

Although the knitted Ultraplast™ wound dressing has good integrity when dry, it becomes weak and loses its integrity when saturated with saline or body fluids. This loss of integrity causes the Ultraplast™ wound dressing to disintegrate while being lifted from a wound (as do other commercial alginate dressings cited above), necessitating that it be picked out in tiny pieces or removed from the wound by irrigation. Because removal by irrigation is a complicated and messy process that requires a substantial degree of skill, users prefer wound dressings that can be lifted from a wound in a single piece.

While we are not aware of any rigorous clinical testing to show any medicinal effect from dressing or packing a wound with an alginate fabric, clinical testing has established that the healing of a wound is enhanced by keeping the wound moist, and alginate dressings and packings admirably retain moisture. Alginate fibers also release well from human tissue.

### Other Prior Art

U.S. Pat. No. 4,704,113 (Schoots) says that two important functions of a surgical or wound dressing are the ability to absorb and hold liquid and the ability to wick and transfer exudate of a wound away from the wound site, but that known dressings which have good absorbtive capability have relatively poor fluid transfer characteristics. The Schoots patent concerns dressings made in accordance with the teachings of U.S. Pat. No. 3,485,706 (Evans) to provide hydroentangled fabrics that "comprise fibers locked into place by fiber interaction to provide a strong cohesive structure which maintains its structural integrity without the need for adhesive binders or filament fusing ... accomplished by first preparing a loose layer of fibers and then passing the layer through an entangler where it is treated with liquid, jetted at a pressure of at least 200 psig. from one or more rows of small orifices" (col. 2, lines 7-29).

Each of the Schoots and Evans patents identifies a large number of fibers that are said to be useful, but neither mentions alginate fibers. Example 1 of Schoots identifies eight fabrics, each made of two different fibers such as a mixture of rayon and polyester fibers.

### Summary of the Invention

The invention provides a method of making an alginate wound dressing comprising the step of processing alginate staple fibers to provide a nonwoven web wherein the web is subjected to a curtain of water streams at high velocity to hydroentangle the nonwoven alginate fiber web into a fabric that has good integrity when saturated with saline fluids.

The alginate wound dressing obtainable by the above mentioned method has sufficient integrity to be lifted in one piece from a wound even though it has become saturated with blood or other saline fluids. This can be accomplished at surprisingly low basis weight, e.g., as low as about 50 g/m². Because of the improved integrity, alginate wound dressings of the present invention can be produced in a variety of basis weights, permitting one to select a dressing of the desired absorbency, thus minimizing the danger of desiccating the wound. Furthermore, when a novel alginate wound dressing of low basis weight is suitable for a particular wound, the dressing can be cost effective in spite of the rather high current cost of alginate fibers.

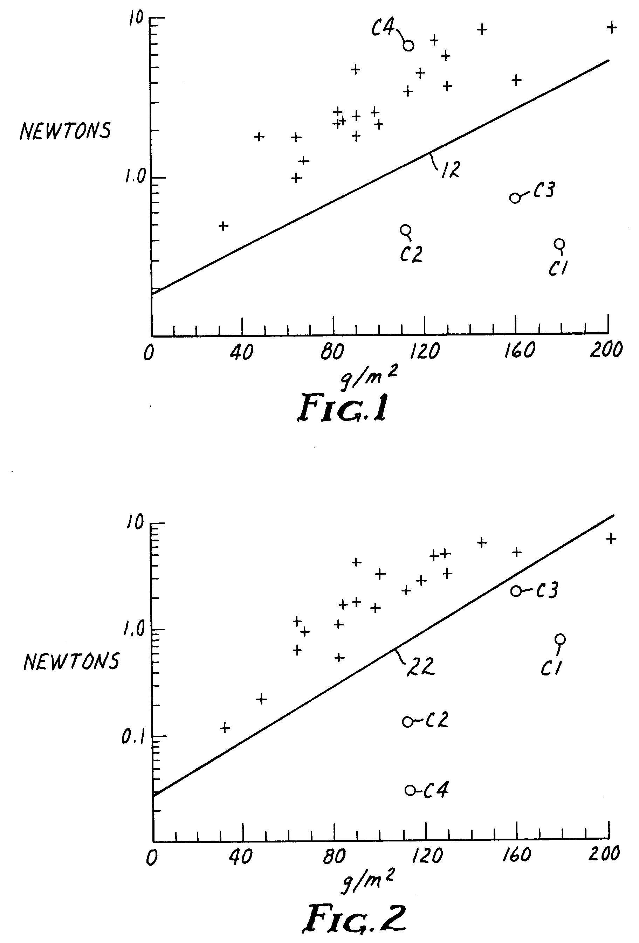

The method particularly produces a nonwoven fabric of alginate staple fibers, which fabric is substantially free from any adhesive binder or of interfusing of fibers at their crossing points. The fabric produced by the method of the invention differs from prior methods in that its fibers are sufficiently entangled that a plot of its tensile strength when dry vs. its basis weight lies above line 12 of Fig. 1 of the drawing when the fabric is dry and lies above line 22 of Fig. 2 when the fabric is saturated with saline water. Furthermore, the fabric has sufficient integrity to permit it to be slit by a continuous process to desired widths, a procedure generally not feasible in the production of the above-mentioned commercial alginate webs because of their poor integrity.

While being hydroentangled, the nonwoven web is preferably supported by an apertured member, such as perforated plate or a screen. By using screens of various sizes, the hydroentangled alginate wound dressings of the invention can have various degrees of openness. When a novel alginate dressing has a highly open structure and is saturated with saline fluids, it has a translucent quality in contact with a wound which permits the wound to be inspected without removing the dressing. Like prior alginate dressings, those of the invention do not swell appreciably in pure water but become highly swelled in saline fluids.

Because of the gelatinous nature of their fibers when saturated with saline fluids, the alginate wound dressings produced by the method when impregnated with therapeutic agents, provide controlled release of those agents into the wound. Useful therapeutic agents include antimicrobials, growth factors, and nutrients.

### Detailed Disclosure

Because alginate fibers are highly absorbent, it may be desirable in some cases to saturate the novel wound dressing with saline water before applying it to a wound, thus minimizing any danger of desiccating the wound. When its basis weight is low, the novel alginate wound dressing preferably includes a small percentage of reinforcing fiber such as rayon to permit it to be handled easily while saturated with saline water.

The novel alginate wound dressings are drapable and easy to use at basis weights up to about 150 g/m², and can be drapable at higher basis weights when they have good openness. However, at basis weights substantially above 150 g/m², the novel dressings may be unduly expensive at the current cost of alginate fibers. Furthermore, alginate wound dressings having higher basis weights would be more likely to desiccate the wound. On the other hand, at basis weights much below 20 g/m², the novel alginate wound dressings may be too weak to be manufactured at commercially viable production rates unless they include reinforcing fibers and probably would need to be die-cut to desired widths instead of being slit.

At basis weights of 60 g/m² or more, the novel alginate fabric produced by the method of the invention, even without reinforcing fibers, has sufficient integrity to permit it to be converted into strips as narrow as about 5 mm using a continuous slitting process. Narrow width dressings are desirable as wound packing materials. In contrast, none of the above-mentioned commercial alginate webs is available in widths less than about 5 cm.

Preferably the alginate staple fibers of the obtained wound dressings are from 2 to 10 cm in length. Longer fiber lengths are difficult to convert into uniform nonwoven webs that can be hydraulically entangled to produce the dressings of the present invention. Staple fibers shorter than 2 cm in length are difficult to convert into nonwoven webs of sufficient integrity to permit them to be hydraulically entangled.

Further improvement to the integrity of entangled alginate wound dressings of the invention can be achieved by incorporating fibers of greater strength such as rayon staple fiber or fibers which interact with the alginate fibers when wet such as chitosan staple fibers. Incorporation can be accomplished by blending the fibers during web formation or by overlaying the nonwoven alginate web with a nonwoven web of the reinforcing fiber prior to hydroentanglement.

It may be desirable to micro-crepe the entangled alginate wound dressings of the invention to enhance their absorptive capability. A suitable technique is taught in the above-cited U.S. Pat. No. 4,704,113.

The alginate wound dressings produced by the method of the invention may be used to pack deep wounds or as absorbent contact layers on shallow wounds. In either case, the alginate dressing should be secured or covered with a material, e.g. a film dressing such as a conventional polyurethane transparent film dressing, a hydrocolloid dressing, a gauze dressing, or a bandage wrap such as gauze or a compression wrap.

### The Drawing

In the drawing:
Fig. 1 plots tensile strength vs. basis weight for representative alginate wound dressings of the invention when dry in comparison to alginate wound dressings of the prior art; and
Fig. 2 plots tensile strength vs. basis weight for the same wound dressings when saturated with saline water.

Referring to Fig. 1, points 10 indicated by "+" represent data of Table I below showing the relationship between tensile strength and basis weight for hydroentangled alginate wound dressings of Examples 1-21 that were tested when dry ("Web Dry Strength" as described below). Points C1, C2, C3 and C4 represent data indicated by "o" to show the same relationship for certain alginate fabrics which are representative of the prior art (Comparative Examples C1, C2, C3 and C4, respectively, identified below). When the fibers of an alginate fabric are sufficiently entangled to achieve the objectives of the invention, a plot of its tensile strength when dry vs. basis weight lies above line 12 of Fig. 1.

In Fig. 2, points 20 indicated by "+" show data of Table I on tensile strength vs. basis weight obtained by testing the same hydroentangled alginate fabrics of Examples 1-21 when saturated with saline water ("Web Wet Strength" as described below). Points C1, C2, C3 and C4 indicated by "o" were obtained by testing Comparative Examples C1-C4. When the fibers of an alginate fabric are sufficiently entangled to achieve the objectives of the invention, a plot of its tensile strength when saturated with saline water vs. basis weight lies above line 22 of Fig. 2.

### Test Methods

### Web Dry Strength

Web Dry Strength is determined by placing a 1.3 cm X 5 cm sample (lengthwise fiber orientation) in an Instron™ tensile tester, having an initial jaw spacing of 2.54 cm, and elongating the sample at a rate of 25.4 cm/minute. The maximum load before break (average of four samples) is recorded.

### Web Wet Strength

Web wet strength is determined as described above for determining the Web Dry Strength except that the web sample is immersed in a 0.9% (w/w) aqueous saline solution for 10 minutes and blotted prior to placing it in the tensile tester.

### Serum Uptake

Serum Uptake is determined by immersing a pre weighed 2.54 cm X 2.54 cm web sample in bovine calf serum for 10 minutes at room temperature and then weighing the sample immediately upon removal from the serum. Reported Serum Uptake values are the average of three samples.

### Nonwoven Web Preparation

Nonwoven webs are prepared by processing calcium alginate fibers (5 cm in length, 2.5-3.0 denier, 16-18% moisture content, available from Courtaulds Fibers Ltd., Coventry U.K.) in either a Rando-Webber Model #12BS or a Hergeth-Hollingsworth Card Type WZM/KS-D2-R2. The webs were hydroentangled as a single ply or as multiple plies having parallel fiber orientation.

Nonwoven calcium alginate fiber webs also are available from Courtaulds Ltd.

### Web Hydroentanglement

Nonwoven webs were converted into alginate wound dressings of the present invention using a Honeycomb Hydraulic Entanglement Flat-Bed Laboratory Test Unit (from Honeycomb, Inc., Biddeford, ME) fitted with a single head. Nonwoven web samples (prepared as described above) were placed on the wire support screen of the unit, pre-wet with water and passed at a rate of 15.25 meters/hour beneath a curtain of pressurized water. Multiple pass entanglement, up to a maximum of four passes on one face, was achieved by reversing the direction of screen travel. The wet web was removed from the support screen and dried in a circulating air oven at about 65°C until the web was dry to the touch (approximately 30 minutes).

### EXAMPLES 1 - 21

Alginate wound dressings of the present invention were prepared according to "Nonwoven Web Preparation" and "Web Hydroentanglement" procedures described above, specific conditions being reported in Tables I and II.

**TABLE I**

| NONWOVEN WEB DESCRIPTION | | | |
|---|---|---|---|
| Example | Nonwoven Web | # of Layers | Basis Weight^{a} (g/m²) |
| 1 | H | 1 | 32 |
| 2 | H | 2 | 64 |
| 3 | H | 3 | 90 |
| 4 | H | 3 | 130 |
| 5 | H | 2 | 64 |
| 6 | H | 3 | 90 |
| 7 | H | 3 | 129 |
| 8 | H | 2 | 84 |
| 9 | H | 3 | 90 |
| 10 | H | 2 | 67 |
| 11 | H | 3 | 112 |
| 12 | H | 3 | 98 |
| 13 | H | 4 | 160 |
| 14 | H | 5 | 100 |
| 15 | R | 1 | 82 |
| 16 | R | 2 | 145 |
| 17 | R | 1 | 124 |
| 18 | C | 10 | 201 |
| 19 | C | 6 | 118 |
| 20 | C | 4 | 82 |
| 21 | C | 2 | 48 |
| H = Hergeth-Hollingsworth R = Rando-Webber C = Carded web obtained from Courtaulds Ltd. | | | |

| | | | |
|---|---|---|---|
| a = Basis weight of hydroentangled web | | | |

**TABLE II**

| HYDROENTANGLEMENT CONDITIONS | | | | | | | | | |
|---|---|---|---|---|---|---|---|---|---|
| Ex. | Wire Mesh^{a} | SIDE 1 WATER PRESSURE (kPA) | | | | Wire Mesh^{a} | SIDE 2 WATER PRESSURE (kPa) | | |
| | | Pass 1 | Pass 2 | Pass 3 | Pass 4 | | Pass 1 | Pass 2 | Pass 3 |
| 1 | 100X100 | 2100 | 4100 | 6200 | -- | 20X20 | 2100 | 4100 | 4100 |
| 2 | 100X100 | 3500 | 6900 | 10300 | -- | 20X20 | 10300 | 3500 | 3500 |
| 3 | 100X100 | 3500 | 6900 | 10300 | -- | 20X20 | 10300 | 3500 | 3500 |
| 4 | 100X100 | 3500 | 6900 | 10300 | -- | 20X20 | 10300 | 3500 | 3500 |
| 5 | 100X100 | 3500 | 6900 | 10300 | -- | 14X14 | 10300 | 3500 | 3500 |
| 6 | 100X100 | 3500 | 6900 | 10300 | -- | 14X14 | 10300 | 3500 | 3500 |
| 7 | 60X60 | 700 | 3500 | 9600 | 9600 | -- | -- | -- | -- |
| 8 | 60X60 | 700 | 3500 | 9600 | 9600 | -- | -- | -- | -- |
| 9 | 14X14 | 10300 | 10300 | 10300 | -- | -- | -- | -- | -- |
| 10 | 14X14 | 700 | 3500 | 9600 | 9600 | -- | -- | -- | -- |
| 11 | 14X14 | 700 | 3500 | 9600 | 9600 | -- | -- | -- | -- |
| 12 | 6X8 | 700 | 3500 | 9600 | 9600 | -- | -- | -- | -- |
| 13 | 6X8 | 700 | 3500 | 9600 | 9600 | -- | -- | -- | -- |
| 14 | 6X8 | 700 | 3500 | 9600 | 9600 | -- | -- | -- | -- |
| 15 | 6X8 | 700 | 3500 | 9600 | 9600 | -- | -- | -- | -- |
| 16 | 6X8 | 700 | 3500 | 9600 | 9600 | -- | -- | -- | -- |
| 17 | 6X8 | 700 | 3500 | 9600 | 9600 | -- | -- | -- | -- |
| 18 | 6X8 | 700 | 3500 | 9600 | 9600 | -- | -- | -- | -- |
| 19 | 6X8 | 700 | 3500 | 9600 | 9600 | -- | -- | -- | -- |
| 20 | 6X8 | 700 | 3500 | 9600 | 9600 | -- | -- | -- | -- |
| 21 | 6X8 | 700 | 3500 | 9600 | 9600 | -- | -- | -- | -- |

| | | | | | | | | | |
|---|---|---|---|---|---|---|---|---|---|
| a = wires/inch X wires/inch | | | | | | | | | |

Results of Web Dry Strength, Web Wet Strength and Serum Uptake testing of these webs are reported in Table III.

### Example 22

A single ply of a rayon staple fiber web (4 cm fiber length, 1.5 denier, 30 g/m² basis weight, formed on a Hollingsworth Card) was overlayed on a single ply of a calcium alginate staple fiber web (5 cm fiber length, 2.8 denier, 80 g/m² basis weight, formed on a Rando Webber) such that the machine direction in the two webs was parallel. The two-ply web structure was hydroentangled using a Honeycomb Entanglement unit fitted with a wire support screen having 6X8 wires per inch, using four passes beneath the pressurized water curtain at 700, 3500, 9600 and 9600 kPa, respectively. The resulting alginate fabric was removed from the screen and heated at about 65°C until dry (approximately 30 minutes). Web Dry Strength and Web Wet Strength data are reported in Table III.

### Comparative Example C1

A carded calcium alginate staple fiber web having a basis weight of 179 g/m² which had been embossed using a heated roll following carding was obtained from Courtaulds Research, Coventry, U.K. to serve as Comparative Example C1.

### Comparative Examples C2-C4

C2 = Sorbsan™ carded web as indentified above.
C3 = Kaltostat™ carded and needle-tacked web as identified above.
C4 = Ultraplast™ knitted styptic gauze as identified above.

Web Dry Strength, Web Wet Strength, and Serum Uptake data for the comparative webs are reported in Table III.

**TABLE III**

| WEB EVALUATION DATA | | | | | | |
|---|---|---|---|---|---|---|
| Example | Basis Weight (g/m²) | Dry Web Strength | | Wet Web Strength | | Serum Uptake (g/cm²) |
| | | (Newtons) | (N/g/m²) | (Newtons) | (N/g/m²) | |
| 1 | 32 | 0.49 | .015 | 0.12 | .004 | 0.07 |
| 2 | 64 | 1.78 | .028 | 1.16 | .018 | 0.13 |
| 3 | 90 | 4.72 | .052 | 4.14 | .046 | 0.17 |
| 4 | 130 | 3.65 | .028 | 3.20 | .025 | 0.16 |
| 5 | 64 | 0.98 | .015 | 0.62 | .010 | 0.13 |
| 6 | 90 | 1.82 | .020 | 1.74 | .019 | 0.14 |
| 7 | 129 | 5.70 | .044 | 4.85 | .036 | 0.13 |
| 8 | 84 | 2.23 | .026 | 1.65 | .020 | 0.12 |
| 9 | 90 | 2.40 | .027 | 4.14 | .046 | 0.18 |
| 10 | 67 | 1.25 | .021 | 0.93 | .014 | 0.14 |
| 11 | 112 | 3.43 | .031 | 2.23 | .020 | 0.15 |
| 12 | 98 | 2.54 | .026 | 1.56 | .016 | 0.18 |
| 13 | 160 | 4.01 | .025 | 5.07 | .032 | 0.23 |
| 14 | 100 | 2.14 | .021 | 3.16 | .032 | 0.18 |
| 15 | 82 | 2.18 | .027 | 0.53 | .006 | 0.14 |
| 16 | 145 | 8.19 | .056 | 6.27 | .043 | 0.17 |
| 17 | 124 | 7.08 | .057 | 4.72 | .038 | 0.17 |
| 18 | 201 | 8.46 | .042 | 6.50 | .032 | 0.18 |
| 19 | 118 | 4.49 | .038 | 2.71 | .023 | 0.16 |
| 20 | 82 | 2.54 | .031 | 1.07 | .013 | 0.13 |
| 21 | 48 | 1.78 | .037 | 0.22 | .005 | 0.11 |
| 22 | 110 | 18.02 | .164 | 13.88 | .126 | ---- |
| C1 | 179 | 0.36 | .002 | 0.76 | .004 | 0.40 |
| C2 | 112 | 0.45 | .004 | 0.13 | .001 | 0.29 |
| C3 | 160 | 0.71 | .004 | 2.14 | .013 | 0.41 |
| C4 | 113 | 6.63 | .059 | 0.03 | .0003 | 0.11 |

In Examples 1-22, web basis weight correlated to Web Wet Strength and Wet Dry Strength in that higher basis weight webs that were otherwise identical generally produced stronger fabrics. However, both the Web Wet Strength and Web Dry Strength of alginate wound dressings of the invention were significantly better than those of comparative Examples C1-C4, even when those of the invention were significantly lower in basis weight.

### Example 23

A blend of 0.1g chitosan staple fiber (4 cm staple length, from Protan Laboratories, Redmond, WA) and 0.9g calcium alginate staple fiber (5 cm length, 2.8 denier, from Courtaulds Fibers Ltd., Coventry, U.K.) was hand carded to provide a 10 cm X 10 cm pad which was hydroentangled by the same procedure used for Example 22. After drying at about 65°C, the entangled web exhibited good integrity.

### Example 24

### Application of Alginate Nonwoven Wound Dressings to Full-Thickness Excisions of Pigs

Two female Yorkshire pigs weighing 35-45 kg were anesthetized using Halothane™, nitrous oxide and oxygen, and their backs were shaved and prepared for surgery. Eight full-thickness excisions approximately 2.5 cm x 2.5 cm in size were made on the backs of each animal. After hemostasis was achieved, two of the wounds on each animal were packed with 4 layers of a Sorbsan™ surgical dressing (Comparative Example C2) which had been cut into 2.5 cm x 2.5 cm squares. Two of the six remaining wounds on each animal were packed with four layers of the hydroentangled alginate wound dressing of Example 8, two with four layers of the dressing of Example 9, and two with four layers of the dressing of Example 13, all of which had been sterilized by ethylene oxide and cut into 2.5 cm x 2.5 cm squares. Each of the alginate-packed wounds was overdressed with a Tegaderm™ transparent film dressing (available from 3M). The pigs were then placed in protective cages to prevent disruption of the dressings and returned to their runs.

Twenty-four hours following surgery, the pigs were anesthetized to allow inspection and changing of the dressings. At that time, all of the alginate dressings had become saturated with exudate, and pooled exudate was evident beneath the Tegaderm™ dressings. After peeling away the Tegaderm™ dressing, all four layers of the alginate dressings in each wound were lifted together or picked out using forceps. The fraction of the wounds from which all four layers of the alginate dressings were lifted out of the wounds in one piece, leaving little or no residue in the wound, is reported in Table IV.

Following irrigation with saline, the wounds were redressed in the same manner as before, each wound being packed with the same type of alginate dressing used previously.

On Days 3, 5 and 7 following surgery (Day 0), the wound dressings were changed using the same procedures as on Day 1, and removability is reported in Table IV.

Histological assessment of biopsies taken on Day 9 following surgery indicated that healing progress was the same for all wounds in the study, regardless of the type of alginate dressing with which they had been dressed.

**Table IV**

| Example | Fraction of Wounds Achieving Removal in One Piece | | | | |
|---|---|---|---|---|---|
| | Day 1 | Day 3 | Day 5 | Day 7 | Totals |
| 8 | 1/4 | 1/4 | 3/4 | 4/4 | 9/16 |
| 9 | 0/4 | 1/4 | 3/4 | 3/4 | 7/16 |
| 13 | 4/4 | 1/4 | 2/4 | 3/4 | 10/16 |
| C2 | 0/4 | 0/4 | 1/4 | 2/4 | 3/16 |

Alginate fabrics of the invention can be made by methods other than as described above. For example, they can be made by creating a nonwoven web of alginate fibers by the method of the above-cited Tong patent and then hydroentangling the fibers of that web.

## Claims

1. Method making an alginate wound dressing comprising the step of processing alginate staple fibers to provide a nonwoven web characterized in that the web is subjected to a curtain of water streams at high velocity to hydroentangle the nonwoven alginate fiber web into a fabric that has good integrity when saturated with saline fluids.

2. Method as defined in preceding claim 1 wherein the nonwoven web is supported by an apertured member while subjected to the curtain of water.

3. A method according to either of claims 1 to 2 and characterized in that said fibers are sufficiently hydroentangled that a plot of the tensile strength of the resulting fabric vs. basis weight lies above line 12 of Fig. 1 of the drawing when the fabric is dry and lies above line 22 of Fig. 2 when the fabric is saturated with saline water.

4. A method according to any one of preceding claims 1 to 3 said fibers being sufficiently entangled that the fabric has a Wet Dry Strength of at least 0.01 N/g/m² and a Web Wet Strength of at least 0.005 N/g/m².

5. A method according to any one of claims 1 to 4 characterized in that the nonwoven fabric obtained has a basis weight of at least 20 g/m².

6. A method according to claim 5 characterized in that fabric obtained has a basis weight of from 50 to 150 g/m².

7. A method according to any one of claims 1 to 6 characterized in that the fabric obtained has a basis weight of at least 60 g/m² and has sufficient integrity to permit it to be converted into strips as narrow as 5 mm using a continuous slitting process.

8. A method according to any one of claims 1 to 7 characterized in that substantially all of the alginate staple fibers are from 2 to 10 cm in length.

9. A method according to any one of claims 1 to 8 characterized in that reinforcing fibers are combined with alginate fibers.

10. A method according to claim 9 further characterized in that the reinforcing fibers are chitosan fibers.

11. A method according to any one of the preceding claims characterized in that it is formed in a highly open structure so that when saturated with saline fluids, and applied to a wound it is sufficiently translucent in contact with the wound to permit the wound to be inspected without removing the dressing.

12. A method according to any one of the preceding claims characterized in that at least one therapeutic agent is added to the fabric.

## Patentansprüche

1. Verfahren zur Herstellung eines Alginatwundverbandes, umfassend den Schritt der Verarbeitung von Alginatstapelfasern zu einem Faservlies, dadurch gekennzeichnet, daß das Vlies mit einem Vorhang von schnell fließenden Wasserstrahlen bearbeitet wird, um das Alginatfaservlies mit Wasser zu einem Gewebe zu verschlingen, das bei Sättigung mit salzigen Flüssigkeiten eine gute Integrität besitzt.

2. Verfahren nach dem vorhergehenden Anspruch 1, bei dem das Vlies auf einem perforierten Element aufliegt, während es mit dem Wasservorhang bearbeitet wird.

3. Verfahren nach einem der Ansprüche 1 bis 2 und dadurch gekennzeichnet, daß die Fasern durch das Wasser soweit ineinander verschlungen sind, daß eine Kurve der Zugfestigkeit des resultierenden Gewebes im Vergleich zum Flächengewicht über der Linie 12 von Fig. 1 der Zeichnung liegt, wenn das Gewebe trocken ist, und über der Linie 22 von Fig. 2 liegt, wenn das Gewebe mit Salzwasser gesättigt ist.

4. Verfahren nach einem der vorhergehenden Ansprüche 1 bis 3, wobei die Fasern soweit ineinander verschlungen sind, daß das Gewebe eine Vlies-Trockenfestigkeit von mindestens 0,01 N/g/m² und eine Vlies-Naßfestigkeit von mindestens 0,005 N/g/m² besitzt.

5. Verfahren nach einem der Ansprüche 1 bis 4, dadurch gekennzeichnet, daß das erhaltene Faservlies ein Flächengewicht von mindestens 20 g/m² besitzt.

6. Verfahren nach Anspruch 5, dadurch gekennzeichnet, daß das erhaltene Faservlies ein Flächengewicht von 50 bis 150 g/m² besitzt.

7. Verfahren nach einem der Ansprüche 1 bis 6, dadurch gekennzeichnet, daß das erhaltene Faservlies ein Flächengewicht von mindestens 60 g/m² besitzt und eine ausreichende Integrität besitzt, um mit einem kontinuierlichen Schneidverfahren zu Streifen verarbeitet werden zu können, die nur 5 mm breit sind.

8. Verfahren nach einem der Ansprüche 1 bis 7, dadurch gekennzeichnet, daß im wesentlichen alle Alginatstapelfasern 2 bis 10 cm lang sind.

9. Verfahren nach einem der Ansprüche 1 bis 8, dadurch gekennzeichnet, daß Verstärkungsfasern mit Alginatfasern kombiniert werden.

10. Verfahren nach Anspruch 9, des weiteren dadurch gekennzeichnet, daß die Verstärkungsfasern Chitosan-Fasern sind.

11. Verfahren nach einem der vorhergehenden Ansprüche, dadurch gekennzeichnet, daß das Vlies in einer sehr offenen Struktur ausgebildet wird, so daß es bei Sättigung mit salzigen Flüssigkeiten, und wenn es auf einer Wunde liegt, in Kontakt mit der Wunde ausreichend durchscheinend ist, so daß die Wunde begutachtet werden kann, ohne den Verband abzunehmen.

12. Verfahren nach einem der vorhergehenden Ansprüche, dadurch gekennzeichnet, daß dem Vlies wenigstens ein Heilmittel zugesetzt ist.

## Revendications

1. Procédé de préparation d'un pansement pour blessures à base d'alginate comprenant l'étape de traitement de fibres coupées d'alginate pour fournir un voile non tissé, caractérisé en ce que le voile est soumis à un rideau de courant d'eau à vitesse élevée pour effectuer un hydro-enchevêtrement du voile de fibres d'alginate non tissé en un textile qui présente une bonne intégrité lorsqu'il est saturé avec des liquides salins.

2. Procédé tel que défini dans la revendication 1 ci-dessus dans lequel le voile non tissé est porte par un élément présentant des ouvertures tout en étant soumis au rideau d'eau.

3. Procédé selon l'une ou l'autre des revendications 1 et 2 et caractérisé en ce que lesdites fibres sont suffisamment hydro-enchevêtrées pour que le graphe de la résistance à la traction du textile résultant en fonction du poids de base se situe au-dessus de la ligne 12 de la figure 1 du dessin lorsque le textile est sec et se situe au-dessus de la ligne 22 de la figure 2 lorsque le textile est saturé avec une eau saline.

4. Procédé selon l'une quelconque des revendications 1 à 3 précédentes lesdites fibres étant suffisamment enchevêtrées pour que le textile ait une résistance du voile à sec d'au moins 0,01 N/g/m² et une résistance du voile à l'état humide d'au moins 0,005 N/g/m².

5. Procédé selon l'une quelconque des revendications 1 à 4, caractérisé en ce que le textile non tissé obtenu a un poids de base d'au moins 20 g/m².

6. Procédé selon la revendication 5, caractérisé en ce que le textile obtenu a un poids de base de 50 à 150 g/m².

7. Procédé selon l'une quelconque des revendications 1 à 6, caractérisé en ce que le textile obtenu a un poids de base d'au moins 60 g/m² et présente une intégrité suffisante pour lui permettre d'être transformé en ruban d'à peine 5 mm de largeur en utilisant un procédé de fendage en continu.

8. Procédé selon l'une quelconque des revendications 1 à 7, caractérisé en ce que sensiblement la totalité des fibres coupées à base d'alginate ont une longueur de 2 à 10 cm.

9. Procédé selon l'une quelconque des revendications 1 à 8, caractérisé en ce que les fibres de renforcement sont combinées avec des fibres d'alginate.

10. Procédé selon la revendication 9, caractérisé en outre en ce que les fibres de renforcement sont des fibres de chitosane.

11. Procédé selon l'une quelconque des revendications précédentes, caractérisé en ce qu'il est réalisé dans une structure hautement ouverte de manière que lorsqu'elle est saturée avec des liquides salins et appliquée à une blessure, elle soit sensiblement translucide au contact avec la blessure pour permettre d'inspecter la blessure sans retirer le pansement.

12. Procédé selon l'une quelconque des revendications précédentes, caractérisé en ce qu'on ajoute au moins un agent thérapeutique au textile.
